# FASCICULE DE BREVET EUROPEEN

(11) **EP 1 071 422 B1**
(45) Date de publication et mention de la délivrance du brevet: **20.02.2008**
(21) Numéro de dépôt: 99915809.0
(22) Date de dépôt: 22.04.1999
(51) Int. Cl.: A61K 31/428, A61P 25/16, A61K 31/198

(54) **ASSOCIATIONS DE RILUZOLE ET DE LEVODOPA POUR LE TRAITEMENT DE LA MALADIE DE PARKINSON**
KOMBINATION VON RILUZOL UND LEVODOPA ZUR BEHANDLUNG VON MORBUS PARKINSON
Association of Riluzole and levodopa for the treatment of Parkinson disease

(30) Priorité: 24.04.1998 FR 9805153
(43) Date de publication de la demande: 31.01.2001
(73) Titulaire: Aventis Pharma S.A., 92160 Antony (FR)
(72) Inventeur: BLANCHARD-BREGEON, Véronique, F-75013 Paris (FR); IMPERATO, Assunta, F-75014 Paris (FR); MOUSSAOUI, Saliha, F-94120 Fontenay sous Bois (FR); OBINU, Marie-Carmen, F-75014 Paris (FR); REIBAUD, Michel, F-94000 Créteil (FR)
(86) Numéro de dépôt international: PCT/FR1999/000953
(87) Numéro de publication internationale: WO 1999/055336

(56) Documents cités:
- RASCOL O. ET AL: "PHARMACOLOGIE CLINIQUE DES DYSKINESIES INDUITES PAR LA L-DOPA CHEZ LES MALADES PARKINSONIENS" THERAPIE (THERAPIE), 53/1 (43-48), February 1998 (1998-02), XP002094840 United Kingdom
- STARR M.S.: "Antiparkinsonian actions of glntamate antagonists - alone and with L-DOPA: A review of evidence and suggestions for possible mechanisms" JOURNAL OF NEURAL TRANSMISSION - PARKINSON'S DISEASE AND DEMENTIA SECTION(J. NEURAL TRANSM. PARKINSON'S DIS. DEMENTIA SECT.), 10/2-3 (141-185), 20 December 1995 (1995-12-20), XP002094841 Austria
- MONTASTRUC J.-L. ET AL: "New directions in the drug treatment of Parkinson's disease" DRUGS AND AGING(DRUGS AGING), 9/3 (169-184),1996, XP002094842 New Zealand
- STARR M.S. ET AL: "Stimulation of basal and L-DOPA-induced motor activity by glutamate antagonists in animal models of Parkinson's disease" NEUROSCIENCE AND BIOBEHAVIORAL REVIEWS(NEUROSCI. BIOBEHAV. REV.), 21/4 (437-446),1997, XP002094843 United Kingdom

## Description

La présente invention concerne une association de la L-DOPA et du riluzole ou d'un sel pharmaceutiquement acceptable de ce composé et l'utilisation de cette association pour le traitement de la maladie de Parkinson.

La maladie de Parkinson est liée à une destruction du locus niger (substance noire) qui a pour conséquence une dégénérescence des neurones dopaminergiques de la voie nigrostriée et donc une diminution massive des taux de la dopamine au niveau du striatum. Pour compenser la déplétion en dopamine qui est consécutive à la dégénérescence des neurones dopaminergiques de la voie nigrostriée chez les patients parkinsoniens, la L-DOPA (3-(3,4-dihydroxyphényl)-L-alanine) ou lévodopa qui est convertie en dopamine par la dopa décarboxylase est utilisée comme traitement symptomatique de la maladie de Parkinson. Après administration orale, la L-DOPA, est massivement décarboxylée au niveau périphérique en dopamine qui ne traverse pas la barrière hématoencéphalique; c'est pourquoi, elle est généralement administrée en association avec un inhibiteur de décarboxylase tel que le bensérazide ou la carbidopa. Ces inhibiteurs de la décarboxylase permettent en effet de réduire par environ 5 la dose de la L-DOPA (Rondot P. et coIL, Pharmacologie Clinique, bases de la thérapeutique, publié par J.-P. Giroud, G. Mathé et G. Meyniel, 2ème édition, Expansion Scientifique Française, 1988, page 1127).

Chez les patients atteints de la maladie de Parkinson, la L-DOPA réduit la sévérité des symptômes tels que la bradykinésie (pauvreté des mouvements), la rigidité musculaire et les tremblements. Cependant le traitement chronique avec la L-DOPA conduit, chez 30% à 80% des patients parkinsoniens, à des effets secondaires et, en particulier, à des dyskinésies (J.G. Nutt, Neurology, 40, 340-345, 1990).

La pharmacologie des dyskinésies induites par la L-DOPA chez les malades parkinsoniens est décrite dans Thérapie 1998, 53, 43-48.

Dans J Neural. Transm, 1995, 141-185, il est revu l'utilisation des produits bloquant ou non les récepteurs NMDA dans le traitement de la maladie de Parkinson.

Dans Drugs Aging, 9/3, (169-184), 1996, il est revu les nouvelles directions pour le traitement de la maladie de Parkinson, la L-DOPA étant toujours le traitement de référence.

Dans Neurosci.Biobehav.Rev. 21/4 (437-446), 1997 décrit l'activité des antagonistes glutamate dans les modèles animaux de la maladie de Parkinson.

Ces dyskinésies sont également reproduites par un traitement chronique avec la L-DOPA chez les marmousets rendus parkinsoniens suite à une injection de la MPTP (1-méthyl-4-phényl-1,2,3,6-tétrahydropyridine), une toxine qui détruit les neurones dopaminergiques de la voie nigrostriée (RK.B. Pearce et coll., Movement Disorders, vol 10, N°6, 731-740, 1995; A. Ekesbo et coll., Neuroreport, 8, 2567-2570, 1997).

Le riluzole (2-amino-6-trifluorométhoxybenzothiazole) est commercialisé pour le traitement de la sclérose latérale amyotrophique. Il est également connu pour son effet neuroprotecteur dans le traitement de la maladie de Parkinson (WO94/15601).

WO9934785 décrit l'association de la L-DOPA et du Riluzole et n'a été publié que le 15.7.99 après le dépôt de la présente demande.

Il a maintenant été trouvé que L'association de riluzole ou un de ses sels pharmaceutiquement acceptables et de L-DOPA améliore l'activité locomotrice des marmousets parkinsoniens et, de plus, prévient les dyskinésies induites par la L-DOPA, lorsqu'ils sont dans un rapport particulier.

L'activité de l'association riluzole et L-DOPA est déterminée selon le protocole suivant : 6 marmousets adultes *(Callithrix jacchus,* Harlan UK) âgés de 25 mois et pesant entre 300 et 350 g sont hébergés dans des cages en acier inoxydable (50 cm de largeur x 20 cm de profondeur x 23 cm de hauteur) avec des portes grillagées, ces cages étant connectées à des plus petites cages (28 x 20 x 23 cm) dans lesquelles les marmousets peuvent dormir. Les animaux sont hébergés dans un environnement contrôlé : température de 24 ± 2°C, humidité de 55%, avec un cycle jour-nuit de 12 heures. Les marmousets ont libre accès à l'eau et ont à disposition chaque jour 35 g de nourriture riche en carbohydrates, protéines et vitamines mélangée avec de l'eau, du lait et du sucre et également des fruits frais.

Tous les animaux reçoivent 3 injections de 2 mg/kg de MPTP par voie sous-cutanée, aux jours 1, 7 et 32. Au jour 32, les animaux sont partagés en 2 groupes. Le groupe 1 (groupe contrôle) reçoit 2 administrations orales de saccharose 10% et le groupe 2 (groupe traité) reçoit 2 administrations orales de riluzole (10 mg/kg) suspendu dans la méthylcellulose 0,5 %, chaque jour jusqu'au jour 104. Au jour 47, les deux groupes d'animaux reçoivent une administration orale de L-DOPA (Modopar^{R} 125 dispersible Roche (L-DOPA (25 mg) + bensérazide (6,25 mg)). Au jour 48, ils reçoivent 2 administrations orales et à partir du jour 49 jusqu'au jour 104, ils reçoivent chaque jour 3 administrations orales de L-DOPA.

### ACTIVITE LOCOMOTRICE

L'activité locomotrice est déterminée en plaçant les animaux dans des cages de test (50 cm x 83cm x 77 cm) qui sont équipées de 3 perchoirs sur lesquels les animaux peuvent sauter ainsi que d'une porte en plexiglass en face de laquelle est placée une caméra. Cette caméra est reliée à un système d'analyseur d'image (Vigie Primates, View Point^{R}) qui est capable de calculer la quantité des mouvements de 8 marmousets, simultanément et indépendamment pendant la durée du test. Le principe de ce système est de quantifier les mouvements des animaux dans la cage dans une fenêtre de temps déterminée (5s). L'image est digitalisée avec une définition 800 x 600 avec 256 niveaux de gris et les changements en pixels d'une image à l'autre sont comptés. Ceci permet de classer l'activité locomotrice en mouvements petits, moyens ou grands. Chaque classe de mouvements est analysée toutes les 10 minutes pendant une période d'une heure. L'activité locomotrice des animaux est mesurée pendant la phase d'exploration c'est-à-dire immédiatement après qu'ils soient placés dans la cage de test.

### SCORE DYSKINESIQUE

Le score dyskinésique est déterminé selon plusieurs paramètres et chacun avec un degré d'intensité différent:

| PARAMETRES | SCORE DYSKINESIQUE | | | | |
|---|---|---|---|---|---|
| | 0 | 1 | 2 | 3 | 4 |
| test du perchoir | normal | léger | modéré | marqué | sévère |
| test d'escalade | normal | léger | modéré | marqué | sévère |
| pattes (antérieures, postérieures) | mouvement amplitude normale | mouvement faible amplitude | mouvement grande amplitude | | |
| posture (dos) | normale | courbure modérée | courbure marquée | | |
| saut | normal | incoordonné | | | |
| motilité | normale | hyperactive | | | |
| chorée | absente | présente | | | |
| dystonie | absente | présente | | | |
| regard | normal | répétitif | | | |
| stéréotypies | absentes | présentes | | | |
| mouvements orolinguaux | absents | présents | | | |
| vocalisation | normale | pour observer | pour communiquer | absente | |

| | | | | | |
|---|---|---|---|---|---|
| Ce score est mesuré aux jours 57, 60, 67 et 104. | | | | | |

L'évaluation est faite 30 mn ou 2 h après la première injection journalière de la L-DOPA (4 h après la solution de riluzole ou de saccharose).

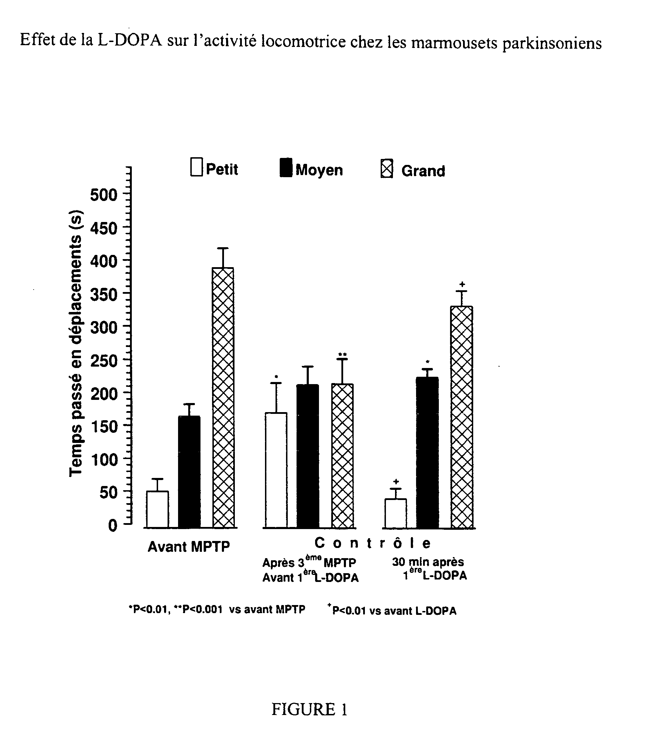

Les résultats sont reportés dans les tableaux 1 à 5 et les figures 1, 2A, 2B et 3 :

Le tableau 1 et la figure 1 montrent que l'administration aiguë de la L-DOPA à la dose de 25 mg/kg, 15 jours après la 3ème injection de MPTP, augmente l'activité locomotrice en diminuant les petits mouvements et en augmentant les grands mouvements. Ces résultats démontrent donc que l'administration aiguë de la L-DOPA améliore l'activité locomotrice.

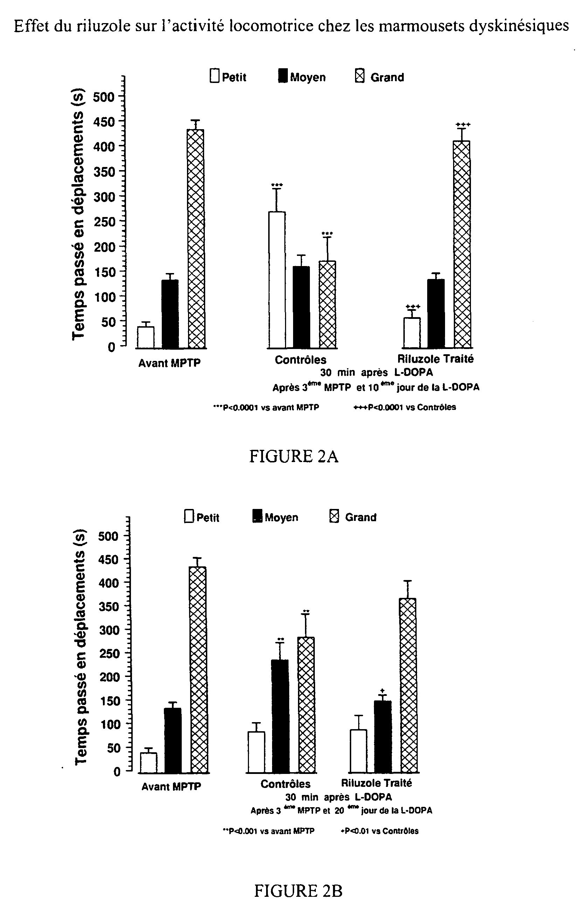

Les tableaux 2, 3 et 4 et les figures 2A et 2B montrent que la L-DOPA, après un traitement répété de 10 jours et de 20 jours, n'a pas ou a peu d'effet bénéfique sur l'activité locomotrice des marmousets parkinsoniens.

Les tableaux 2, 3 et 4 et les figures 2A et 2B montrent aussi que le riluzole à la dose de 10 mg/kg, par voie orale, 2 fois par jour pendant 72 jours est capable d'améliorer l'activité locomotrice et ceci est observé aussi bien après 10 jours qu'après 20 jours de traitement avec la L-DOPA.

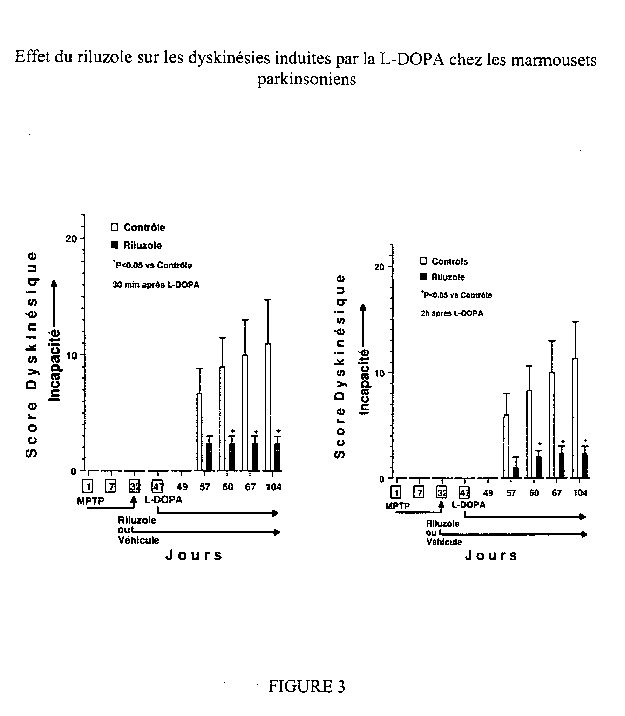

Le tableau 5 et la figure 3 montrent que les administrations répétées de la L-DOPA (25 mg/kg, par voie orale, 3 fois par jour) produisent des dyskinésies observées aux jours 57, 60, 67 et 104, avec un effet maximal au jour 104.

Le tableau 5 et la figure 3 montrent aussi que le riluzole (à la dose de 10 mg/kg, par voie orale, 2 fois par jour) réduit les dyskinésies induites par la L-DOPA et ceci est observé du jour 57 au jour 104.

**TABLEAU 1 : Effet de la L-DOPA sur l'activité locomotrice des marmousets parkinsoniens**

| | | ***Groupe 1 (saccharose)*** | | | | | | | | |
|---|---|---|---|---|---|---|---|---|---|---|
| | Marmouset n°. | **1** | **2** | **3** | **1** | **2** | **3** | **1** | **2** | **3** |
| Classe des mouvements | Intervalle de temps des mesures | ***avant MPTP*** | | | ***avant la 1ère administration de la administration de la L-DOPA*** | | | ***30 mn après la 1ère administration de la administration de la L-DOPA*** | | |
| Mouvements petits | 1 - 10 | 290 | 15 | 10 | 90 | 175 | 50 | 5 | 305 | 20 |
| | 11 - 20 | 25 | 0 | 40 | 135 | 35 | 115 | 25 | 35 | 10 |
| | 21 - 30 | 215 | 20 | 5 | 85 | 95 | 485 | 5 | 20 | 0 |
| | 31 - 40 | 40 | 0 | 0 | 45 | 0 | 455 | 15 | 60 | 55 |
| | 41 - 50 | 60 | 35 | 0 | 45 | 5 | 560 | 45 | 15 | 10 |
| | 51 - 60 | 120 | 5 | 5 | 115 | 50 | 545 | 30 | 35 | 35 |
| | Moyenne ± E.S.M.. | **49 ± 19** | | | **171 ±46** * | | | **40 ± 16** + | | |
| Mouvements moyens | 1 - 10 | 140 | 185 | 60 | 255 | 285 | 375 | 235 | 270 | 250 |
| | 11 - 20 | 165 | 75 | 60 | 260 | 270 | 475 | 190 | 290 | 240 |
| | 21-30 | 200 | 245 | 165 | 275 | 175 | 75 | 225 | 205 | 110 |
| | 31-40 | 270 | 115 | 15 | 310 | 130 | 130 | 355 | 205 | 255 |
| | 41 - 50 | 270 | 265 | 125 | 280 | 95 | 40 | 260 | 165 | 165 |
| | 51 - 60 | 280 | 150 | 155 | 255 | 110 | 55 | 240 | 200 | 215 |
| | Moyenne ± E.S.M.. | **163 ± 19** | | | **214 ± 28** | | | **226 ± 13** * | | |
| Mouvements grands | 1 - 10 | 175 | 405 | 535 | 260 | 145 | 180 | 365 | 30 | 335 |
| | 11 - 20 | 410 | 525 | 500 | 205 | 295 | 10 | 385 | 275 | 350 |
| | 21 - 30 | 185 | 335 | 430 | 240 | 330 | 40 | 370 | 375 | 490 |
| | 31 -40 | 290 | 485 | 585 | 245 | 470 | 15 | 230 | 335 | 290 |
| | 41 - 50 | 270 | 300 | 475 | 275 | 500 | 0 | 295 | 420 | 425 |
| | 51 - 60 | 200 | 445 | 440 | 230 | 440 | 0 | 330 | 365 | 350 |
| | Moyenne ± E.S.M.. | **388 ± 30** | | | **216 ± 38** ** | | | **334 ± 23** + | | |

| | | | | | | | | | | |
|---|---|---|---|---|---|---|---|---|---|---|
| Différence significative *versus* la valeur avant MPTP : * P≤0,01 ** P≤0,001 (test Student); différence significative *versus* la valeur avant la L-DOPA : + P≤0,01 (test Student) | | | | | | | | | | |

**TABLEAU 2 : Effet du riluzole sur l'activité locomotrice chez les marmousets dyskinésiques**

| ***Avant MPTP*** | | | | | | | |
|---|---|---|---|---|---|---|---|
| Classe des mouvements | Intervalle de temps de mesure (mn) | **Temps passé en déplacements** | | | | | |
| | | ***Groupe 1*** | | | ***Groupe 2*** | | |
| | Marmouset n°. | **1** | **2** | **3** | **4** | **5** | **6** |
| Mouvements petits | 1 - 10 | 290 | 15 | 10 | 35 | 10 | 5 |
| | 11 - 20 | 25 | 0 | 40 | 25 | 0 | 0 |
| | 21 - 30 | 215 | 20 | 5 | 70 | 25 | 40 |
| | 31 - 40 | 40 | 0 | 0 | 45 | 0 | 5 |
| | 41 - 50 | 60 | 35 | 0 | 105 | 0 | 10 |
| | 51 - 60 | 120 | 5 | 5 | 25 | 55 | 5 |
| | Moyenne ± E.S.M. | **37 ± 10** | | | | | |
| Mouvements moyens | 1 - 10 | 140 | 185 | 60 | 50 | 60 | 35 |
| | 11-20 | 165 | 75 | 60 | 45 | 85 | 35 |
| | 21 - 30 | 200 | 245 | 165 | 105 | 95 | 150 |
| | 31 - 40 | 270 | 115 | 15 | 80 | 60 | 80 |
| | 41 - 50 | 270 | 265 | 125 | 160 | 60 | 145 |
| | 51 - 60 | 280 | 150 | 155 | 245 | 215 | 95 |
| | Moyenne ± E.S.M. | **132 ± 13** | | | | | |
| Mouvements grands | 1 - 10 | 175 | 405 | 535 | 520 | 535 | 565 |
| | 11 - 20 | 410 | 525 | 500 | 530 | 515 | 565 |
| | 21 - 30 | 185 | 335 | 430 | 425 | 480 | 410 |
| | 31 - 40 | 290 | 485 | 585 | 475 | 540 | 515 |
| | 41 - 50 | 270 | 300 | 475 | 335 | 540 | 445 |
| | 51 - 60 | 200 | 445 | 440 | 330 | 330 | 500 |
| | Moyenne ± E.S.M. | **432 ± 19** | | | | | |

**TABLEAU 3 : Effet du riluzole sur l'activité locomotrice chez les marmousets dyskinésiques**

| ***10 jours après la L-DOPA*** | | | | | | | |
|---|---|---|---|---|---|---|---|
| Classe des mouvements | Intervalle de temps des mesures (mn) | **Temps passé en déplacements** | | | | | |
| | | (évalué 30 mn après la 1ère administration journalière de la L-DOPA) | | | | | |
| | | ***Groupe 1*** | | | ***Groupe 2*** | | |
| | | ***(saccharose)*** | | | ***riluzole (2 x 10 mg*/*kg)*** | | |
| | Marmouset n°. | **1** | **2** | **3** | **4** | **5** | **6** |
| Mouvements petits | 1 - 10 | 5 | 510 | 375 | 5 | 5 | 5 |
| | 11 - 20 | 0 | 500 | 295 | 0 | 5 | 45 |
| | 21 - 30 | 0 | 560 | 365 | 0 | 125 | 50 |
| | 31 - 40 | 10 | 485 | 370 | 0 | 145 | 25 |
| | 41 - 50 | 10 | 465 | 230 | 30 | 195 | 40 |
| | 51 - 60 | 205 | 225 | 240 | 35 | 210 | 100 |
| | Moyenne ± E.S.M.. | **269 ± 47***** | | | **57 ± 16⁺⁺⁺** | | |
| Mouvements moyens | 1 - 10 | 115 | 55 | 190 | 40 | 105 | 80 |
| | 11 - 20 | 70 | 70 | 295 | 35 | 140 | 185 |
| | 21 - 30 | 100 | 40 | 235 | 60 | 120 | 115 |
| | 31 - 40 | 155 | 35 | 175 | 140 | 215 | 190 |
| | 41 - 50 | 155 | 115 | 370 | 155 | 205 | 175 |
| | 51-60 | 165 | 305 | 235 | 135 | 125 | 190 |
| | Moyenne ± E.S.M.. | **160 ± 23** | | | **134 ± 13** | | |
| Mouvements grands | 1 - 10 | 485 | 40 | 40 | 560 | 495 | 520 |
| | 11 - 20 | 530 | 30 | 10 | 565 | 455 | 370 |
| | 21 - 30 | 500 | 0 | 0 | 540 | 355 | 435 |
| | 31 - 40 | 435 | 80 | 55 | 460 | 240 | 385 |
| | 41 - 50 | 435 | 20 | 0 | 415 | 200 | 385 |
| | 51 - 60 | 230 | 70 | 125 | 430 | 265 | 310 |
| | Moyenne ± E.S.M.. | **171 ± 48***** | | | **410 ± 25⁺⁺⁺** | | |

| | | | | | | | |
|---|---|---|---|---|---|---|---|
| Différence significative *versus* valeur avant MPTP : *** P ≤ 0,0001 (test de Student) Différence significative *versus* valeur de contrôle ; ⁺⁺⁺ P ≤ 0,0001 (test de Student). | | | | | | | |

**TABLEAU 4 : Effet du riluzole sur l'activité locomotrice chez les marmousets dyskinésiques**

| ***20 jours après la L-DOPA*** | | | | | | | |
|---|---|---|---|---|---|---|---|
| Classe des mouvements | Intervalle de temps des mesures (mn) | **Temps passé en déplacement** | | | | | |
| | | (évalué 30 mn après la 1ère administration journalière de la L-DOPA) | | | | | |
| | | ***Groupe 1*** | | | ***Groupe 2*** | | |
| | | ***(saccharose)*** | | | ***riluzole (2 x 10 mg*/*kg)*** | | |
| | Marmouset n°. | **1** | **2** | **3** | **4** | **5** | **6** |
| Mouvements petits | 1 - 10 | 105 | 5 | 45 | 5 | 130 | 35 |
| | 11 - 20 | 40 | 0 | 45 | 5 | 395 | 5 |
| | 21 - 30 | 120 | 10 | 105 | 0 | 170 | 20 |
| | 31-40 | 125 | 0 | 65 | 5 | 275 | 15 |
| | 41 - 50 | 130 | 15 | 265 | 0 | 135 | 15 |
| | 51 - 60 | 185 | 0 | 230 | 0 | 345 | 15 |
| | Moyenne ± E.S.M.. | **83 ± 19** | | | **87 ± 30** | | |
| Mouvements moyens | 1 - 10 | 360 | 0 | 475 | 155 | 140 | 80 |
| | 11 - 20 | 260 | 20 | 475 | 255 | 160 | 105 |
| | 21 - 30 | 225 | 35 | 400 | 175 | 250 | 100 |
| | 31-40 | 325 | 55 | 300 | 180 | 175 | 80 |
| | 41 - 50 | 265 | 95 | 280 | 95 | 220 | 125 |
| | 51 - 60 | 315 | 30 | 315 | 90 | 180 | 105 |
| | Moyenne ± E.S.M.. | **235 ± 37**** | | | **148 ± 13⁺** | | |
| Mouvements grands | 1 - 10 | 140 | 600 | 85 | 445 | 335 | 490 |
| | 11 - 20 | 300 | 580 | 80 | 340 | 45 | 490 |
| | 21 - 30 | 255 | 555 | 95 | 425 | 180 | 480 |
| | 31 - 40 | 150 | 545 | 235 | 415 | 150 | 505 |
| | 41 - 50 | 205 | 490 | 55 | 505 | 245 | 460 |
| | 51 - 60 | 100 | 570 | 55 | 510 | 75 | 480 |
| | Moyenne ± E.S.M.. | **283 ± 50**** | | | **365 ± 37** | | |

| | | | | | | | |
|---|---|---|---|---|---|---|---|
| Différence significative *versus* valeur avant MPTP : ** P ≤ 0,001 (test de Student) Différence significative *versus* valeur de contrôle : ⁺ P≤ 0,01 (test de Student). | | | | | | | |

**TABLEAU 5 : Effet du riluzole sur les marmousets dyskinésiques**

| Jour après la 1^{ère} injection de MPTP | **Score dyskinésique total** | | | | | | | |
|---|---|---|---|---|---|---|---|---|
| | (évalué 30 mn après la 1 ère administration journalière de la L-DOPA) | | | | | | | |
| | ***Groupe* 1: *contrôles*** | | | | ***Groupe 2: riluzole*** | | | |
| | ***(saccharose)*** | | | | ***(2 x 10 mg*/*kg p. o.)*** | | | |
| | Valeurs individuelles | | | Moyenne | Valeurs individuelles | | | Moyenne |
| Marmouset n°. | **1** | **2** | **3** | ± E. S. M.. | **4** | **5** | **6** | ±E.S.M.. |
| 57 | 4 | 11 | 5 | 6,7 ± 2,1 | 3 | 3 | 0 | 2,3 ± 0,7 |
| 60 | 4 | 11 | 12 | 9,0 ± 2,5 | 3 | 3 | 1 | 2,3 ± 0,7⁺ |
| 67 | 4 | 14 | 12 | 10 ± 3,0 | 3 | 3 | 1 | 2,3 ± 0,7⁺ |
| 104 | | 17 | 12 | 11 ± 3,8 | 1 | 3 | 3 | 2,3 ± 0,7⁺' |
| | | | | | | | | |

| Jour après la 1^{ère} injection de MPTP | **Score dyskinésique total** | | | | | | | |
|---|---|---|---|---|---|---|---|---|
| | (évalué 2 h après la 1ère administration journalière de la L-DOPA) | | | | | | | |
| | ***Groupe 1: contrôles*** | | | | ***Groupe 2: riluzole*** | | | |
| | ***(saccharose)*** | | | | ***(2 x 10 mg*/*kg p. o.)*** | | | |
| | Valeurs individuelles | | | Moyenne | Valeurs individuelles | | | Moyenne |
| Marmouset n°. | **1** | **2** | **3** | ± E.S.M.. | **4 5 6** | | | ± E.S.M.. |
| 57 | 3 | 10 | 5 | 6.0 ± 2,1 | 3 | 3 | 0 | 2,.0 ± 1,0 |
| 60 | 4 | 9 | 12 | 8,3 ± 2,3 | 2 | 3 | 1 | 2,0 ± 0,6⁺ |
| 67 | 4 | 13 | 13 | 10 ± 3,0 | 3 | 3 | 1 | 213 ± 0,7⁺ |
| 104 | 5 | 17 | 12 | 11 ± 3.0 | 1 | 3 | 3 | 2.3 ± 0.7⁺ |

| | | | | | | | | |
|---|---|---|---|---|---|---|---|---|
| Différence significative intergroupe *versus* valeur de contrôle égale : ⁺ P≤ 0,05 (test de Student). | | | | | | | | |

En conclusion, ces résultats démontrent que, d'une part, la L-DOPA après une administration aiguë a amélioré l'activité locomotrice en diminuant les petits déplacements, et en augmentant les grands déplacements des marmousets parkinsoniens. Au contraire, la L-DOPA, après un traitement répété, a non seulement produit peu ou pas d'effet sur l'activité locomotrice, mais a en plus produit des effets secondaires, les dyskinésies chez les marmousets parkinsoniens.

D'autre part, ces résultats montrent que chez les marmousets parkinsoniens, le riluzole améliore l'activité locomotrice et prévient le développement des dyskinésies induites par le traitement chronique de la L-DOPA. L'association riluzole et L-DOPA a donc un double effet bénéfique chez les marmousets parkinsoniens, en améliorant l'activité locomotrice et en diminuant les effets secondaires, les dyskinésies, induits par la L-DOPA.

Le riluzole ou un de ses sels pharmaceutiquement acceptables et la L-DOPA peuvent être administrés sous forme d'une combinaison et éventuellement associés à tout autre produit pharmaceutiquement compatible, pouvant être inerte ou physiologiquement actif.

Le riluzole ou un de ses sels pharmaceutiquement acceptables et la L-DOPA peuvent également être administrés séparément ou d'une manière étalée dans le temps de façon à obtenir le maximum d'efficacité.

Ainsi au sens de la présente invention, les associations ne sont pas uniquement limitées à celles qui sont obtenues par mélange physique des constituants mais aussi à celles qui permettent une administration séparée qui peut être simultanée ou étalée dans le temps.

On peut également ajouter à cette association un inhibiteur de la décarboxylase tel que le bensérazide ou la carbidopa.

Dans les associations selon l'invention, on utilise 10 à 400 parties en poids de riluzole pour 100 à 6000 parties en poids de L-DOPA et, de préférence, 200 à 4000 parties en poids de L-DOPA ou l'équivalent de cette quantité lorsque la L-DOPA est en mélange avec un inhibiteur de décarboxylase. Généralement, la quantité de L-DOPA lorsque celle-ci est en mélange avec un inhibiteur de L-DOPA est de 50 à 1500 parties en poids.

Lorsque l'inhibiteur de décarboxylase est le bensérazide, on en utilise généralemnt une quantité en poids 2 à 6 fois moindre que la quantité de L-DOPA et plus particulièrement 4 fois moindre que la quantité de L-DOPA.

Lorsque l'inhibiteur de décarboxylase est la carbidopa, on en utilise généralement une quantité en poids 2 à 15 fois moindre que la quantité de L-DOPA et plus particulièrement 4 à 10 fois moindre que la quantité de L-DOPA.

L'association peut être employée par voie orale, parentérale ou rectale.

Comme sels pharmaceutiquement acceptables du riluzole peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, iséthionate, théophilline-acétate, salicylate, phénolphtalinate, méthylène-bis-6-oxynaphtoate ou des dérivés de substitution de ces dérivés.

Comme compositions solides pour administration orale, peuvent être utilisés des comprimés, des pilules, des poudres (capsules de gélatine, cachets) ou des granulés. Dans ces compositions, les principes actifs sont mélangés à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semisynthétiques ou des polyéthylèneglycols.

La présente invention concerne également la méthode de traitement des patients parkinsoniens qui consiste à administrer au patient une association L-DOPA et riluzole ou un de ses sels pharmaceutiquement acceptable et éventuellement un inhibiteur de décarboxylase soit simultanément soit séparément soit de manière étalée dans le temps.

Les doses dépendent de l'effet recherché, de la durée du traitement et de la voie d'administration utilisée; elles sont généralement de 10 à 400 mg par jour par voie orale pour un adulte avec des doses unitaires allant de 10 à 200 mg de riluzole et de 100 à 6000 mg et de préférence 200 à 4000 mg par jour par voie orale pour un adulte avec des doses unitaires de 100 à 250 mg de L-DOPA ou l'équivalent de cette dose lorsque la L-DOPA est administrée avec un inhibiteur de décarboxylase. Ainsi lorsque la L-DOPA est administrée avec un inhibiteur de décarboxylase la dose de L-DOPA est généralement de 50 à 1500 mg par jour par voie orale.

Lorsque l'inhibiteur de décarboxylase est le bensérazide, il est préférable d'administrer par jour, par voie orale, pour un adulte, 10 à 400 mg de riluzole, 50 à 1500 mg de L-DOPA et une quantité de bensérazide en poids 2 à 6 fois moindre et particulièrement 4 fois moindre que la quantité en poids de L-DOPA.

Lorsque l'inhibiteur de décarboxylase est la carbidopa, il est préférable d'administrer par jour, par voie orale, pour un adulte, 10 et 400 mg de riluzole, 50 à 1500 mg de L-DOPA et une quantité de carbidopa en poids 2 à 15 fois moindre et particulièrement 4 à 10 fois moindre que la quantité en poids de L-DOPA.

D'une façon générale, le médecin déterminera la posologie appropriée en fonction de l'âge, du poids et de tous les autres facteurs propres au sujet à traiter.

## Revendications

1. Association de 10 à 400 parties en poids de riluzole ou d'un sel pharmaceutiquement acceptable de ce dernier avec 100 à 6000 parties en poids de lévodopa.

2. Association selon la revendication 1 dans laquelle on utilise 10 à 400 parties en poids de riluzole pour 200 à 4000 parties en poids de lévodopa.

3. Association de la lévodopa, d'un inhibiteur de décarboxylase et du riluzole ou d'un sel pharmaceutiquement acceptable de ce dernier.

4. Association selon la revendication 3 dans laquelle l'inhibiteur de décarboxylase est le bensérazide ou la carbidopa.

5. Association selon la revendication 3 dans laquelle on utilise 10 à 400 parties en poids de riluzole ou d'un sel pharmaceutiquement acceptable de ce dernier pour 50 à 1500 parties en poids de lévodopa en mélange avec un inhibiteur de décarboxylase.

6. Association selon la revendication 5 dans laquelle on utilise le bensérazide comme inhibiteur de décarboxylase.

7. Association selon la revendication 5 dans laquelle on utilise la carbidopa comme inhibiteur de décarboxylase.

8. Association selon l'une des revendications 1 à 7 comme préparation combinée pour une utilisation simultanée, séparée ou étalée dans le temps.

9. Utilisation d'association selon l'une des revendications 1 à 8 pour la préparation d'un médicament utile dans le traitement de la maladie de Parkinson.

10. Utilisation d'association selon l'une des revendications 1 à 8 pour la préparation d'un médicament utile dans la prévention et le traitement des dyskinésies induites par la lévodopa.

## Claims

1. Combination of 10 to 400 parts by weight of riluzole or a pharmaceutically acceptable salt of the latter with 100 to 6000 parts by weight of levodopa.

2. Combination according to Claim 1 in which 10 to 400 parts by weight of riluzole are used per 200 to 4000 parts by weight of levodopa.

3. Combination of levodopa, a decarboxylase inhibitor and riluzole or a pharmaceutically accepted salt of the latter.

4. Combination according to Claim 3 in which the decarboxylase inhibitor is benserazide or carbidopa.

5. Combination according to Claim 3 in which 10 to 400 parts by weight of riluzole or a pharmaceutically acceptable salt of the latter are used per 50 to 1500 parts by weight of levodopa as a mixture with a decarboxylase inhibitor.

6. Combination according to Claim 5 in which benserazide is used as a decarboxylase inhibitor.

7. Combination according to Claim 5 in which carbidopa is used as a decarboxylase inhibitor.

8. Combination according to one of Claims 1 to 7 as a combined preparation for simultaneous use, separate use or use which is spread out in time.

9. Use of a combination according to one of Claims 1 to 8 for the preparation of a medicament useful in the treatment of Parkinson's disease.

10. Use of a combination according to one of Claims 1 to 8 for the preparation of a medicament useful in the prevention and treatment of dyskinesias induced by levodopa.

## Patentansprüche

1. Kombination von 10 bis 400 Gewichtsteilen Riluzol oder einem pharmazeutisch annehmbaren Salz davon mit 100 bis 6000 Gewichtsteilen Levodopa.

2. Kombination nach Anspruch 1, bei der man 10 bis 400 Gewichtsteile Riluzol pro 200 bis 4000 Gewichtsteile Levodopa verwendet.

3. Kombination von Levodopa, einem Decarboxylasehemmer und Riluzol oder einem pharmazeutisch annehmbaren Salz davon.

4. Kombination nach Anspruch 3, in der der Decarboxylasehemmer Benserazid oder Carbidopa ist.

5. Kombination nach Anspruch 3, bei der man 10 bis 400 Gewichtsteile Riluzol oder ein pharmazeutisch annehmbares Salz davon pro 50 bis 1500 Gewichtsteile Levodopa in Abmischung mit einem Decarboxylasehemmer einsetzt.

6. Kombination nach Anspruch 5, bei der man Benserazid als Decarboxylasehemmer einsetzt.

7. Kombination nach Anspruch 5, bei der man Carbidopa als Decarboxylasehemmer einsetzt.

8. Kombination nach einem der Ansprüche 1 bis 7 als Kombinationspräparat für die zeitlich gestaffelte, getrennte oder gleichzeitige Verwendung.

9. Verwendung einer Kombination nach einem der Ansprüche 1 bis 8 zur Herstellung eines Arzneimittels für die Behandlung von Morbus Parkinson.

10. Verwendung einer Kombination nach einem der Ansprüche 1 bis 8 zur Herstellung eines Arzneimittels für die Vorbeugung und die Behandlung von levodopainduzierten motorischen Fehlfunktionen.
